# EUROPEAN PATENT APPLICATION

(11) **EP 3 839 515 A1**
(43) Date of publication of application: **23.06.2021**
(21) Application number: 19217673.3
(22) Date of filing: 18.12.2019
(51) Int. Cl.: G01N 35/00

(54) **METHOD AND DEVICES FOR ASSESSING THE SUITABILITY OF A SAMPLE TUBE FOR USE IN A LABORATORY AUTOMATION SYSTEM**

(71) Applicant: Roche Diagnostics GmbH, 68305 Mannheim (DE); F. Hoffmann-La Roche AG, 4070 Basel (CH)
(72) Inventor: Fleischmann, Timo, 71332 Waiblingen (DE)
(74) Representative: Altmann Stößel Dick Patentanwälte PartG mbB

(57) **Abstract**

A method of assessing the suitability of at least one sample tube (116) for use in a laboratory automation system (114) is disclosed. The method comprises:
i) capturing at least one image of at least a part of the sample tube (116) by using a camera (120) of a mobile device (112);
ii) obtaining at least one first item of identification information of the sample tube (116) by analyzing the image;
iii) retrieving at least one second item of identification information of the sample tube (116); and
iv) evaluating the first and second items of identification information and assigning the sample tube (116) to at least one suitability category.

Further, a mobile device (112) and an assessment system (110) for assessing the suitability of at least one sample tube (116) for use in a laboratory automation system (114) are disclosed.

## Description

### Technical Field

The present invention refers to a method of assessing the suitability of at least one sample tube for use in a laboratory automation system by using a camera of a mobile device. The invention further relates to an assessment system for assessing the suitability of at least one sample tube for use in a laboratory automation system. Further, the present invention refers to a mobile device having at least one camera, to a computer program and a computer-readable storage medium for performing the method. The methods, devices, computer programs and computer-readable storage media specifically may be used in the field of medical or chemical laboratories, for example in order to assess the suitability of a sample tube for use in an analytical or in a pre-analytical laboratory system. Other fields of application of the present invention, however, are feasible.

### Background art

In the field of medical or chemical laboratories, a wide variety of samples, such as blood, blood serum or plasma, urine or chemical substances, are handled in many different sample tube types. In many cases, the suitability of sample tubes for use in a specific laboratory automation system has to be assessed before use. In some cases, a laboratory automation system autonomously checks the suitability of a specific sample tube for use in the system.

Thus, devices and methods known to the skilled person which make use of laboratory automation systems comprising, as an example, a camera system to optically identify the sample tubes used in the system. As an example, WO 2015/191702 A1 discloses methods and systems for detecting properties of sample tubes in a laboratory environment, including a drawer vision system that can be trained and calibrated. Images of a tube tray captured by at least one camera are analyzed to extract image patches that allow a processor to automatically determine if a tube slot is occupied, if the tube has a cap. The processor can be trained using a random forest technique and a plurality of training image patches. Cameras can be calibrated using a three-dimensional calibration target that can be inserted into the drawer.

EP 3223019 A1 discloses a method of establishing a sample tube set which is adapted to be processed by a laboratory automation system, comprising selecting a sample tube set comprising several sample tube types by selecting a plurality of different sample tube types from an assortment of available sample tube types. The method further comprises obtaining a parameter of distribution for at least one detection parameter of each sample tube type comprised in said sample tube set, wherein the parameter of distribution comprises information regarding a distribution of previously detected reference data values of the at least one detection parameter. The method further comprises determining whether or not the laboratory automation system is capable of correctly identifying each sample tube type comprised in said sample tube set by comparing the parameter of distribution for the at least one detection parameter of each sample tube type comprised in said sample tube set with the parameter of distribution for the at least one detection parameter of all the other sample tube types comprised in said sample tube set. The method also comprises indicating that the selected sample tube set is approved for being processed by the laboratory automation system, if it is determined that the laboratory automation system is capable of correctly identifying each sample tube type comprised in said sample tube set, or proposing at least one conflict remediation, if it is determined that the laboratory automation system is not capable of correctly identifying each sample tube type comprised in said sample tube set.

EP 3357842 A1 discloses a laboratory automation system for processing sample containers containing laboratory samples and/or for processing the samples, comprising: a digital camera being adapted to take an image of the sample container together with a calibration element, wherein the image comprises image data related to the sample container and image data related to the calibration element, and an image processing device being adapted to determine geometrical properties of the sample container depending on the image data related to the sample container and the image data related to the calibration element.

EP 2148205 B1 discloses a method and laboratory system for handling sample tubes and an image analyzing unit. The laboratory system comprises a transfer unit for transferring incoming primary racks (PR) containing sample tubes, an image analyzing unit, and an identification and allocation unit, the method to handle laboratory sample tubes in a laboratory system comprising the steps of: transferring, by means of the transfer unit, an incoming primary rack containing sample tubes to the image analyzing unit; determining, in the image analyzing unit, geometry parameters of at least one sample tube contained in the primary rack by means of image analyzing; in the identification and allocation unit, comparing for each sample tube the determined geometry parameters with predetermined geometry criteria and identifying whether the sample tube's geometry fulfils the predetermined criteria; in case of fulfilment, categorizing the sample tube as system conform; otherwise categorizing the sample tube as non-system conform; wherein each sample tube identified as system conform is entered into further processing and each sample tube identified as non-system conform is entered into error processing.

US 9417253 B2 discloses a specimen processing system comprising: a specimen measuring section for measuring specimens accommodated in specimen containers; a transport section for transporting specimen containers to the specimen measuring section; a specimen container collect section for collecting specimen containers; an obtainer for obtaining shape information on specimen containers or state information on specimens accommodated in specimen containers; a supply judger configured for determining whether specimen containers are to be supplied to the specimen measuring section on the basis of the result obtained by the obtainer; and a delivery section for delivering specimen containers, which are determined to be supplied to the specimen measuring section by the supply judger, toward the transport section, and delivering specimen containers, which are determined not to be supplied to the specimen measuring section by the supply judger, toward the specimen container collect section. A specimen container classifying apparatus is also disclosed.

US 2016/0018427 A1 discloses a method and a system for combining container identification data from a container inspection unit that analyzes a container containing a liquid with liquid level detection raw data from a liquid level detection unit that analyzes the container containing the liquid and a liquid level detection result is generated. The liquid level detection result is crosschecked with additional data from the container inspection unit. The result can be used to plan a route for the container in the laboratory automation system.

EP 3408640 A1 discloses methods and apparatus adapted to identify a specimen container from multiple lateral views.

US 6599476 B1 discloses a pathology distribution system provided for automated sample container distribution. The system comprises a loading station for loading samples in primary containers of different types, a sample handling station for receiving the containers and identifying the container types and samples therein, and a container distribution station for distributing the containers in areas or racks in the distribution station marked for analyzing processes prescribed for the samples therein.

Despite the advantages achieved by the known methods and devices, specifically in the field of automated optical inspection of the sample tubes, several technical challenges remain. Specifically, the possibility of assessing the suitability of a sample tube for use in a laboratory automation system independent and apart from the system and in advance of use of the sample tube would be desirable. A manual qualification process of unknown sample tubes by qualified technicians in many cases is ineffective as well as time- and cost-consuming.

### Problem to be solved

It is therefore desirable to provide a method and devices which at least partially address the above-mentioned technical challenges of known methods and devices of similar kind. Specifically, a method and devices shall be proposed which allow for assessing the suitability of a sample tube for use in a laboratory automation system in advance, in a simple and cost-effective manner.

### Summary

This problem is addressed by a method and an assessment system for assessing the suitability of at least one sample tube for use in a laboratory automation system, further by a mobile device, a computer program and a computer-readable storage medium with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, a method of assessing the suitability of at least one sample tube for use in a laboratory automation system is disclosed. The method comprises the following steps which specifically may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed.

The method comprises:
i) capturing at least one image of at least a part of the sample tube by using a camera of a mobile device;
ii) obtaining at least one first item of identification information of the sample tube by analyzing the image;
iii) retrieving at least one second item of identification information of the sample tube, specifically by the mobile device, and
iv) evaluating the first and second items of identification information and assigning the sample tube to at least one suitability category.

The term "assessing the suitability for use" as used herein is a broad term and is to be given its ordinary and customary meaning to a person of ordinary skill in the art and is not to be limited to a special or customized meaning. The term specifically may refer, without limitation, to the process of providing at least one item of information on whether or not at least one element or process is suited for being used in conjunction with another element and/or another process, for the purpose of achieving a specific result. The item of information may be or may comprise a digital information, such as "suited" or "not suited". Additionally or alternatively, the item of information may be or may comprise a degree of suitability, indicating the extent to which the element or process is suited. Thus, in the context of assessing the suitability of at least one sample tube for use in a laboratory automation system, the assessment specifically may refer, without limitation, to a qualitative and/or quantitative assessment of the suitability of at least one sample tube in an arbitrary and/or a specific laboratory automation system (LAS). The LAS may be configured to handle a sample tube with certain specifications. For example, these specifications may refer to one or more of: the sample tube's geometry, the sample tube's ground shape, the cap type, specifically the cap geometry, the cap color, the cap sealing type, the cap material, and/or the sample tube's material. The result of the assessment may comprise information about the at least one suitability category of the sample tube regarding the specific LAS. For example, the result may comprise information whether or not the sample tube is compatible with the specific LAS. As another example, the result of the assessment may also comprise information whether the sample tube is of a known type.

The term "suitability category" as used herein is a broad term and is to be given its ordinary and customary meaning to a person of ordinary skill in the art and is not to be limited to a special or customized meaning. The term specifically may refer, without limitation, to a classification with regard to the usability of a sample tube in a LAS. The usability of a sample tube may thereby depend on specific requirements of the LAS the sample tube is used for. Thus, the suitability category specifically may reflect and/or comprise the at least one information generated in the assessment. As an example, the suitability category may reflect whether the sample tube is suited for use or is unsuited for use with the LAS or, otherwise, whether the suitability may not be decided and whether additional information on the suitability may have to be retrieved. At least two suitability categories may be defined or pre-defined.

The term "sample tube" as used herein is a broad term and is to be given its ordinary and customary meaning to a person of ordinary skill in the art and is not to be limited to a special or customized meaning. The term specifically may refer, without limitation, to a container which is adapted to one or more of contain, store or transport a sample to be analyzed in the LAS. Further, the shape of the container can be such that a tube is formed, such as a cylindrical tube, e.g. a cylindrical tube having a circular and/or polygonal cross-section. Other types or forms of the container are also possible. The sample tube may comprise a tube bottom, a tube body and a cap, including a sealing of the cap. The tube bottom may be configured to confine the sample tube at the sample tube's lower end. The tube body may be configured to form the shape of the sample tube. The cap may be configured to reversibly close up the sample tube at the sample tube's upper end by using a mechanism of a specific sealing type. For example, the sample tube's cap sealing may comprise one or more of a screw type, a rubber type, a hemogard type or a push type. The upper and lower end of the sample tube may be defined by the way of usage of the sample tube.

Further, the term "laboratory automation system" (LAS) as used herein is a broad term and is to be given its ordinary and customary meaning to a person of ordinary skill in the art and is not to be limited to a special or customized meaning. The term specifically may refer, without limitation, to a system which is configured to handle sample tubes automatically, specifically a system which is adapted to process sample tubes and their enclosed samples autonomously and/or fully or partially automatically. The laboratory in which the LAS is used may be for example a clinical laboratory, a forensic laboratory or a blood bank. The LAS, as an example, may comprise at least one actuator configured for handling the at least one sample tube, such as configured for handling a plurality of sample tubes, e.g. sequentially or in parallel. The actuator, as an example, may be configured for automatically moving the at least one sample tube, such as through a plurality of handling stations within the system. As an example, the actuator may be or may comprise one or more of a robot arm, a conveying system or a carrousel. Other actuators are known and may be used. The LAS may further comprise one or more handling stations for handling and/or processing one or more samples contained in the at least one sample tube. As an example, one or more stations for adding components to the sample, one or more stations for heating the sample or one or more stations for sample separation such as by centrifuging may be provided. The LAS specifically may be a pre-analytical system, specifically for preparing one or more samples for subsequent sample analysis.

The term "sample" as used herein is a broad term and is to be given its ordinary and customary meaning to a person of ordinary skill in the art and is not to be limited to a special or customized meaning. The term specifically may refer, without limitation, to an aliquot of a substance such as a chemical or biological compound. Specifically, the sample may be or may comprise at least one biological specimen, such as one or more of: blood; blood serum; blood plasma; urine; saliva. Additionally or alternatively, the sample may be or may comprise a chemical substance or compound and/or a reagent.

The method, as outlined above, comprises, in step i), capturing at least one image of at least a part of the sample tube by using the camera of the mobile device. The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as one or more of a mobile phone, a cell phone, a smartphone, a tablet computer, a notebook. Additionally or alternatively, as will be outlined in further detail below, the mobile device may also refer to a tablet computer or another type of portable computer having at least one camera. The mobile device specifically may be portable, such as by having dimensions of less than 5.000 cm³, and/or by having a weight of less than 3 kg.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images.

The term "image" as used herein is a broad term and is to be given its ordinary and customary meaning to a person of ordinary skill in the art and is not to be limited to a special or customized meaning. The term specifically may refer, without limitation, to data recorded by using a camera, such as a plurality of electronic readings from the imaging device, such as the pixels of the camera chip. Thus, the image may be or may comprise at least one array of information values, such as an array of grey scale values and/or color information values. The image may be a single color image or a multi-color or colored image.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smartphones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. A larger number of color values is also feasible, such as four color values for each pixel. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used. The color sensitivities of the pixels may be generated by color filters or by appropriate intrinsic sensitivities of the sensor elements used in the camera pixels. These techniques are generally known to the skilled person.

The term "capturing at least one image" as used herein is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by the user action or may automatically be initiated, e.g. once the presence of the at least one object within a field of view and/or within a predetermined sector of the field of view of the camera is automatically detected. These automatic image acquisition techniques are known e.g. in the field of automatic barcode readers, such as from automatic barcode reading apps. The capturing of the images may take place, as an example, by acquiring a stream or "live stream" of images with the camera, wherein one or more of the images, automatically or by user interaction such as pushing a button, are stored and used as the at least one first image or the at least one second image, respectively. The image acquisition may be supported by a processor of the mobile device, and the storing of the images may take place in a data storage device of the mobile device.

The method, as outlined above, comprises, in step ii), obtaining at least one first item of identification information of the sample tube by analyzing the image. The term "item of identification information" as used herein is a broad term and is to be given its ordinary and customary meaning to a person of ordinary skill in the art and is not to be limited to a special or customized meaning. The term specifically may refer, without limitation, to an arbitrary characteristic which can be used to identify a sample tube. For example, an item of identification information may describe a geometry, a color, a material and/or a functional feature of the sample tube. The terms "first" and "second" are used for the purpose of nomenclature, only, without ranking or numbering these items of identification information and without giving any preferences. Further, this numbering does not refer to a specific chronological order of the information or to a specific number of information. Specifically, one or more, more specifically two or more than two items of identification may be obtained for both, the first and second item of identification information. The items of identification information may be obtained via different ways. One possible way of obtaining an item of identification information is by means of image analysis. Another possible way of obtaining an item of identification information is by retrieving the information via an interface of the mobile device, as will be outlined in further detail below.

The term "analyzing the image", also referred to as "image analysis", as used herein is a broad term and is to be given its ordinary and customary meaning to a person of ordinary skill in the art and is not to be limited to a special or customized meaning. The term specifically may refer, without limitation, to an arbitrary process for deriving information from the image. Specifically, in context of step ii), the information obtained by analyzing the image contain at least one first item of identification information. The process of image analysis may be implemented by using a computer vision process. Image analysis techniques are generally known to the skilled person. Thus, as an example, pattern recognition techniques may be used which are generally known, e.g. for detecting the geometry of the sample tube or the like. Other image analysis steps or processes may be used in addition or alternatively. The result of the analyzing of the image may be or may comprise a numerical result and/or another type of information which may directly be used as the first item of identification information and/or which may be used for generating, by further processing, the at least one first item of identification information.

The term "retrieving", as used herein and as specifically used in the context of step iii), is a broad term and is to be given its ordinary and customary meaning to a person of ordinary skill in the art and is not to be limited to a special or customized meaning. The term specifically may refer, without limitation, to the process of actively or passively obtaining one or more of an object and an item of information, specifically data. The retrieving may be initiated by the retrieving entity or by an external entity. The object or item of information to be retrieved may be obtained, as an example, via one or more interfaces, such as a data interface and/or a user interface. As an example, a user may provide the at least one second item of identification information of the sample tube by manually inserting the information, such as in a process of guided data entry, e.g. via one or more queries initiated automatically by the mobile device, e.g. by an App running on the mobile device.

The method, as further outlined above, in step iv), comprises evaluating the first and second item of identification information and assigning the sample tube to at least one suitability category. The term "evaluating" as used herein is a broad term and is to be given its ordinary and customary meaning to a person of ordinary skill in the art and is not to be limited to a special or customized meaning. The term specifically may refer, without limitation, to an operation of processing one or more input information / data and thereby obtaining an evaluation result or evaluation quantity, such as one or more of an information, a variable or a parameter. For example, in context of step iv), the first and second item of identification information may be evaluated in order to generate at least one item of information allowing for assigning the sample tube to the at least one suitability category. Examples will be given in further detail below.

The second item of identification information specifically may comprise information other than information obtained by image analysis. Thus, as an example, the second item of information may be or may comprise information which is not derived or derivable from the image analysis such as one or more items of information selected from the group consisting of information provided by the user, information provided by a data base, information provided by another device communicating with the mobile device. As an example, the second item of identification information may be at least partially retrieved via at least one interface of the mobile device. The interface may comprise at least one of a data interface and a user interface, specifically a graphical user interface. The mobile device may prompt the user to input the second item of identification information or at least a part thereof. Thus, the second item of identification information may be at least partially retrieved by a user input, specifically by a manual user input, such as via a user interface of the mobile device. Further, the second item of identification information may also be at least partially retrieved via a software application running on the mobile device, the software application requesting a user of the mobile device to input the second item of identification information, specifically via an input menu, more specifically via having the user select from a plurality of predetermined selectable items. Other ways of retrieving the at least one second item of identification information may be feasible alternatively or additionally.

The term "interface of the mobile device" as used herein is a broad term and is to be given its ordinary and customary meaning to a person of ordinary skill in the art and is not to be limited to a special or customized meaning. The term specifically may refer, without limitation, to a feature of the mobile device which is configured to interact with its environment, such as for the purpose of unidirectionally or bidirectionally exchanging information, such as for exchange of one or more of data or commands. For example, the interface of the mobile device may be configured to share information with a user and to receive information by the user. The interface of the mobile device may be a feature to interact visually with a user, such as a display, or a feature to interact acoustically with the user. The interface, as an example, may comprise one or more of: a user interface, such as a graphical user interface; a data interface, such as a wireless and/or a wirebound data interface.

The term "software application" as used herein is a broad term and is to be given its ordinary and customary meaning to a person of ordinary skill in the art and is not to be limited to a special or customized meaning. The term specifically may refer, without limitation, to a computer program running on a computing unit in order to perform a specific operation. The software application may provide a specific functionality to the computing unit. The computing unit may comprise a computer, a mobile device, a server system.

The term "input menu / user input" as used herein is a broad term and is to be given its ordinary and customary meaning to a person of ordinary skill in the art and is not to be limited to a special or customized meaning. The term specifically may refer, without limitation, to a functionality of the software application to record information provided by the user. The input menu may be part of a graphical user interface for retrieving information from the user. Further, the term "user" as used herein is a broad term and is to be given its ordinary and customary meaning to a person of ordinary skill in the art and is not to be limited to a special or customized meaning. The term specifically may refer, without limitation, to a person who operates the mobile device, specifically the software application running on the mobile device. The user, as an example, may be a service technician qualified to handle sample tubes and/or a LAS or may be an unexperienced user.

As outlined above, step iv) comprises evaluating the first and second item identification information and assigning the sample tube to at least one suitability category. This step may comprise combining the first and second item of identification information to obtain at least one combined item of identification information of the sample tube, wherein in step iv), the combined item of identification information is evaluated. For example, the combined item of identification information may comprise a serial number or an article number of the sample tube. Based on this combined item of identification information, a suitability category may be assigned to the sample tube. Additionally or alternatively, the at least one combined item of identification information may be or may comprise a vector and/or an array of items of information, such as an array comprising both the at least one first item of identification information and the at least one second item of identification information. The combined item of identification information may then be used for further processing, such as for identifying the sample tube and/or for determining the suitability category.

The method may further comprise:
v) providing the suitability category via at least one interface, specifically via at least one interface of the mobile device, more specifically via at least one display of the mobile device.
Thus, as an example, the software application running on the mobile device may, besides optionally retrieving the at least one second item of identification information, be configured for providing the suitability category via the at least one interface of the mobile device, such as via the at least one graphical user interface, specifically for displaying the suitability on at least one display of the mobile device. It shall be noted that other options are also feasible. Thus, as an example, the suitability category may also, additionally or alternatively, be brought to user's attention by other means, such as by audio means and/or haptic means, and/or may be stored in at least one data storage device and/or may be transmitted to at least one other device, such as via the at least one interface, such as to the LAS. The method may also comprise outputting a warning, such as one or more of a visual warning, an audio warning or a haptic warning, such as in case the suitability category may indicate that the sample tube is unsuited for use with the LAS.

As outlined above, the method may comprise capturing and/or determining the sample tube's geometry, such as in step ii). For this purpose, as an example, step i) may comprise using at least one calibration element, wherein the image may comprise at least a part of the calibration element. Thus, as an example, the capturing of the at least one image may take place such that the image shows at least a part of the sample tube and, additionally, at least a part of the calibration element. The calibration element may comprise at least one geometrical reference marker. Further, the image of the at least one part of the calibration element may be used to obtain at least one item of geometrical information of at least a part of the sample tube. The item of geometrical identification information may comprise at least one of: a height of the sample tube, specifically one or more of a height of the sample tube including a cap, a height of a tube body of the sample tube without a cap and a height of the sample tube's cap, more specifically one or more of a height of the sample tube's cap without a nose, a height of the sample tube's cap with nose and a height of the nose of a sample tube's cap; a width of the sample tube, specifically one or more of a width of a sample tube body of the sample tube and a width of a cap of the sample tube; an inner diameter of the sample tube, specifically one or more of an inner diameter of a sample tube body of the sample tube, more specifically one or more of an inner diameter at a predefined position above the sample tube's bottom and an inner diameter at a predefined position below the sample tube's upper edge, and an inner diameter of a cap of the sample tube; an outer diameter of the sample tube, specifically one or more of an outer diameter of a sample tube body of the sample tube, more specifically one or more of an outer diameter at a predefined position above the sample tube's bottom and an outer diameter at a predefined position below the sample tube's upper edge, and an outer diameter of a cap of the sample tube; a shape of at least a part of the sample tube, specifically one or more of a shape of a tube bottom of the sample tube and a shape of a cap of the sample tube; a geometry of a tube bottom of the sample tube, specifically one or more of a radius of a round bottom of the sample tube and a length of a flat bottom of the sample tube; a geometry of a thread of the sample tube, specifically one or more of an outer diameter of a thread of the sample tube, a length of a thread of the sample tube and a number of threads of a thread of the sample tube; an angle of the sample tube's body, specifically one or more of a left angle of the sample tube's body and a right angle of the sample tube's body.

The method of assessing the suitability of the sample tube may also take into account a color information of the sample tube. Therefore, the calibration element may comprise at least one color reference. The image of the at least one part of the calibration element may be used to obtain at least one item of color identification information of at least a part of the sample tube. For example, the item of color identification information may comprise at least one item of color information on a cap of the sample tube. Both items of identification information, the geometrical and color information of the sample tube may be determined by the image analysis in step ii). Therefore, the obtained first item of identification information may comprise at least one of an item of geometrical identification information and/or an item of color identification information.

The term "calibration element" as used herein is a broad term and is to be given its ordinary and customary meaning to a person of ordinary skill in the art and is not to be limited to a special or customized meaning. The term specifically may refer, without limitation, to an object of known geometrical and/or color properties which is used to calibrate one or more of geometrical, dimensional or color information derived from the image. The calibration element, as an example, may be or may comprise at least one substrate, such as a cardboard substrate or a plastic substrate. The substrate may provide calibration information, such as by having one or more of predetermined and known dimensions, color or the like. Additionally or alternatively, the one or more geometrical patterns and/or color patterns may be applied to the substrate, such as printed onto at least one surface of the substrate. As example, the calibration element may comprise at least one printed calibration pattern having known dimensions and/or having known color properties, such as having known R, G or B color coordinated. The calibration element may be used such that the sample tube is placed onto the calibration element when performing step i), such that both the sample tube and the calibration element are at least partially visible in the image. Additionally or alternatively, the at least one calibration element may be placed next to the sample tube, such that both the sample tube and the calibration element are at least partially visible in the image. The calibration of the image may, as an example, be performed by comparing the pixel data of the calibration element and the pixel data of the sample tube. The calibration element may also comprise a colored element to calibrate a color scale of the image. For this purpose, the RGB color intensities measured by a color camera may be used. Thus, generally, in step ii), the analysis of the image may take into account at least one item of reference information derived from the image of the calibration element, such as at least one item of geometrical reference information and at least one item of color reference information.

Further, as outlined above, the method comprises retrieving the at least one second item of identification information in step iii). The second item of identification information may comprise at least one of: an item of material identification information, an item of functional identification information, an item of geometrical identification information. The item of material identification information specifically may comprise at least one of an information on a material of a tube body of the sample tube and/or information on a material of a cap of the sample tube. The item of functional identification information specifically may comprise information on a cap sealing type of a cap of the sample tube. The item of geometrical identification information specifically may comprise information on a tube geometry of at least a part of the sample tube, specifically on one or more of a geometry of a tube bottom of the sample tube and a cap of the sample tube.

As outlined above, the method comprises in step iv), evaluating the first and second item of identification information. Step iv) specifically may be performed by using at least one algorithm, specifically a machine learning algorithm, more specifically a machine learning classifier, for example a computer vision algorithm. Further in step iv), assigning of the sample tube to at least one suitability category may be performed by using at least one sample tube database. The suitability category may therefore indicate whether or not the sample tube is of a type already listed in the sample tube database. Thus, the suitability category may comprise at least one category from the group consisting of: "known and compatible", "unknown, but compatible" and "not compatible". The suitability category may indicate whether or not the sample tube is suitable for being used with the laboratory automation system. In case the sample tube may be of a type not yet listed in the sample tube database, the sample tube database may be updated. Specifically, the sample tube database may be updated by using at least one result of step iv).

The term "sample tube database" as used herein is a broad term and is to be given its ordinary and customary meaning to a person of ordinary skill in the art and is not to be limited to a special or customized meaning. The term specifically may refer, without limitation, to an electronic data management system which is configured to handle a specific data structure, wherein the data refer to sample tubes. The specific data structure may be determined by a database model. The sample tube database may be configured as a qualified sample tube list. For example, the sample tube database may be or may comprise a register which relates one or more items of identification information to a specific sample tube. The sample tube database may, thus, comprise data on a plurality of sample tube types and additional data, such as data referring to properties of the sample tube types. Further, the sample tube database may comprise suitability information on the sample tube types, such as the suitability category and/or other suitability information relating to the sample tube types listed in the sample tube database.

The term "algorithm" as used herein is a broad term and is to be given its ordinary and customary meaning to a person of ordinary skill in the art and is not to be limited to a special or customized meaning. The term specifically may refer, without limitation, to one or more rules for processing input data. For example, the algorithm may be a machine learning algorithm which is able to learn the rule of processing by processing data, for example by processing training data.

The mobile device specifically, as outlined above, may comprise at least one processor. The term "processor" as generally used in this description is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like. The processor specifically may be configured, such as by software programming, for performing one or more evaluation operations.

Further, the processor may be adapted to perform step ii) and optionally step iii). The processor may be adapted to automatically initiate the capturing of the image in step i), specifically by recognizing if the sample tube is visible in a field of view of the camera.

Step iv) of the method may be at least partially performed by a backend server remote from the mobile device, specifically by a cloud server. Therefore, the method may comprise transmitting the data obtained in one or more of steps i), ii), and iii) from the mobile device to the backend server. The data obtained in one or both of steps i) and iii) may be recorded via a software application running on the mobile device. The data transmission specifically may take place via one or more wireless and/or wirebound interfaces or networks, such as via the internet.

The term "backend server" as used herein is a broad term and is to be given its ordinary and customary meaning to a person of ordinary skill in the art and is not to be limited to a special or customized meaning. The term specifically may refer, without limitation, to a device which is capable of performing one or more operations with the data to be processed. Specifically, the server may comprise at least one processor, which may be programmed, by appropriate software, for performing one or more operations with the data to be processed. The at least one backend server may be at least one cloud server.

In a further aspect of the present invention, an assessment system for assessing the suitability of at least one sample tube for use in a laboratory automation system is disclosed. The assessment system comprises at least one mobile device having at least one camera, wherein the mobile device is configured, specifically by software programming, for capturing at least one image of at least a part of the sample tube by using the camera. The assessment system is configured, specifically by software programming, for performing the method of assessing the suitability of a sample tube for use in a LAS according to the present invention, such as according to any one of the embodiments disclosed above and/or according to any one of the embodiments disclosed in further detail below.

Therefore, the assessment system may further comprise, as outlined above in the context of the method, at least one backend server remote from the mobile device, specifically a cloud server, wherein the backend server may be configured for data exchange with the mobile device, wherein the backend server may be configured, specifically by software programming, for at least partially performing step iv). As outlined above, the assessment system comprises the mobile phone which may be further configured, specifically by software programming, for performing at least one of steps ii) and iii) of the method of assessing the suitability of a sample tube for use in a LAS.

For further definitions and options of the assessment system, reference may be made to the description of the method in one or more of the embodiments given above and/or according to any one of the embodiments described in further detail below.

In a further aspect of the present invention, a mobile device is disclosed, specifically for use in an assessment system according to the present invention, such as according to any one of the embodiments disclosed above and/or according to any one of the embodiments disclosed in further detail below. The mobile device is configured, specifically by software programming, for performing at least steps i), ii) and iii) of the method according to the present invention, such as according to any one of the embodiments disclosed above and/or according to any one of the embodiments disclosed in further detail below. The mobile device may be further configured for transmitting data obtained in one or more of steps i), ii), and iii) to a backend server.

For further definitions and options of the mobile device, reference may be made to the description of the method in one or more of the embodiments given above and/or according to any one of the embodiments described in further detail below.

In a further aspect, a computer program is disclosed, comprising instructions which, when the program is executed by at least one processor of the assessment system according to the present invention, such as according to any one of the embodiments disclosed above and/or according to any one of the embodiments disclosed in further detail below, cause the processor to carry out the method according to the present invention, such as according to any one of the embodiments disclosed above and/or according to any one of the embodiments disclosed in further detail below. Similarly, a computer-readable storage medium is disclosed, comprising instructions which, when executed by at least one processor of the assessment system according to the present invention, such as according to any one of the embodiments disclosed above and/or according to any one of the embodiments disclosed in further detail below, cause the processor to carry out the method according to the present invention, such as according to any one of the embodiments disclosed above and/or according to any one of the embodiments disclosed in further detail below.

As used herein, the term "computer-readable storage medium" specifically may refer to a non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

The computer program may also be embodied as a computer program product. As used herein, a computer program product may refer to the program as a tradable product. The product may generally exist in an arbitrary format, such as in a paper format, or on a computer-readable data carrier and/or on a computer-readable storage medium. Specifically, the computer program product may be distributed over a data network.

The methods and devices according to the present invention may provide a large number of advantages over similar methods and devices known in the art. Thus, compared to methods and devices known in the art, the methods and devices described herein may provide the possibility of assessing the suitability of at least one sample tube for use in a laboratory automation system using a mobile device having a camera. Specifically, an immediate evaluation of the sample tube independent and apart from the laboratory automation system may improve flexibility and handling of such an assessment method. Thereby, known sample tubes can be activated immediately for use in a laboratory automation system. If possible, unknown tubes can be qualified immediately and/or potential restrictions can be imposed. Therefore, manual handling and/or manual decanting of sample tubes used in the laboratory automation system may become obsolete.

Further, in case the sample tube has been classified as being unsuitable for the laboratory automation system, it can be thought immediately of an alternative. Thus, the method may enable the consulting staff to evaluate the suitability of a sample tube independent and apart from the laboratory automation system. Advices about sample tubes which are already qualified and can be used immediately may be given by non-technician staff. Thus, this method may provide for an easy and comprehensive way of assessing the suitability of sample tubes for use in a laboratory automation system.

As another advantage which may result from the method using a mobile device, an assessment of the sample tubes by technical staff such as staff from a research and development allowance may become redundant or obsolete. The evaluation may be done automatically by the software application running on the mobile device. Therefore, the method and the assessment system may enable a time- and cost-efficient evaluation of the sample tube.

Further, the method and assessment system of assessing the suitability of the sample tubes may allow a cost-effective and eco-friendly assessment by using a mobile device, since the sample tube to be checked doesn't need unnecessary shipment to an inspection site.

As outlined above, the method may fully or partially be computer-implemented. Thus, the method may fully or partially be embodied by a software application, such as a software application running on the mobile device, and/or by a software combination comprising at least one software package or software application running on the mobile device and at least one further software package running on the backend server. As indicated above, at least one or more of steps i), ii), and iii) may be computer-implemented or computer-supported by a software application running on the mobile device. Step iv) may be software implemented by one or more of the software application running on the mobile device and/or by a software running on the backend server. The remaining and optional steps may also fully or partially be software implemented, as the skilled person will recognize.

Summarizing and without excluding further possible embodiments, the following embodiments may be envisaged:
Embodiment 1: A method of assessing the suitability of at least one sample tube for use in a laboratory automation system, comprising:
   i) capturing at least one image of at least a part of the sample tube by using a camera of a mobile device;
   ii) obtaining at least one first item of identification information of the sample tube by analyzing the image;
   iii) retrieving at least one second item of identification information of the sample tube, specifically by the mobile device; and
   iv) evaluating the first and second items of identification information and assigning the sample tube to at least one suitability category.
Embodiment 2: The method according to the preceding embodiment, wherein the second item of identification information comprises information other than information obtained by image analysis.
Embodiment 3: The method according to any one of the preceding embodiments, wherein the second item of identification information is at least partially retrieved via at least one interface of the mobile device.
Embodiment 4: The method according to the preceding embodiment, wherein the interface comprises at least one of a data interface and a user interface, specifically a graphical user interface.
Embodiment 5: The method according to any one of the preceding embodiments, wherein the second item of identification information is at least partially retrieved by a user input, specifically by a manual user input.
Embodiment 6: The method according to the preceding embodiment, wherein the mobile device prompts the user to input the second item of identification information.
Embodiment 7: The method according to any one of the two preceding embodiments, wherein the second item of identification information is at least partially retrieved via a software application running on the mobile device, the software application requesting a user of the mobile device to input the second item of identification information, specifically via an input menu, more specifically via having the user select from a plurality of predetermined selectable items.
Embodiment 8: The method according to any one of the preceding embodiments, wherein step iv) comprises combining the first and second items of identification information to obtain at least one combined item of identification information of the sample tube, wherein, in step iv), the combined item of identification information is evaluated.
Embodiment 9: The method according to any one of the preceding embodiments, further comprising:
   v) providing the suitability category via at least one interface, specifically via at least one interface of the mobile device, more specifically via at least one display of the mobile device.
Embodiment 10: The method according to the preceding embodiment, wherein step i) comprises using at least one calibration element, wherein the image comprises at least a part of the calibration element.
Embodiment 11: The method according to the preceding embodiment, wherein the calibration element comprises at least one geometrical reference marker.
Embodiment 12: The method according to the preceding embodiment, wherein the image of the at least one part of the calibration element is used to obtain at least one item of geometrical information of at least a part of the sample tube.
Embodiment 13: The method according to any one of the three preceding embodiments, wherein the calibration element comprises at least one color reference.
Embodiment 14: The method according to the preceding embodiment, wherein the image of the at least one part of the calibration element is used to obtain at least one item of color information of at least a part of the sample tube.
Embodiment 15: The method according to any one of the preceding embodiments, wherein step iv) is performed by using an algorithm, specifically a machine learning algorithm, more specifically a machine learning classifier.
Embodiment 16: The method according to any one of the preceding embodiments, wherein the assigning of the sample tube to at least one suitability category in step iv) is performed by using at least one sample tube database.
Embodiment 17: The method according to the preceding embodiment, wherein the suitability category indicates whether or not the sample tube is of a type already listed in the sample tube database.
Embodiment 18: The method according to any one of the two preceding embodiments, wherein the suitability category comprises at least one category from the group consisting of: "known and compatible", "unknown, but compatible" and "not compatible".
Embodiment 19: The method according to any one of the three preceding embodiments, wherein the sample tube database is updated by using at least one result of step iv).
Embodiment 20: The method according to the preceding embodiment, wherein the sample tube database is updated in case the sample tube is of a type not yet listed in the sample tube database.
Embodiment 21: The method according to any one of the preceding embodiments, wherein the suitability category indicates whether or not the sample tube is suitable for being used with the laboratory automation system.
Embodiment 22: The method according to any one of the preceding embodiments, wherein the first item of identification information comprises at least one of: an item of geometrical identification information; an item of color identification information.
Embodiment 23: The method according to the preceding embodiment, wherein the item of geometrical identification information comprises at least one of: a height of the sample tube, specifically one or more of a height of the sample tube including a cap, a height of a tube body of the sample tube without a cap and a height of the sample tube's cap, more specifically one or more of a height of the sample tube's cap without a nose, a height of the sample tube's cap with nose and a height of the nose of a sample tube's cap; a width of the sample tube, specifically one or more of a width of a sample tube body of the sample tube and a width of a cap of the sample tube; an inner diameter of the sample tube, specifically one or more of an inner diameter of a sample tube body of the sample tube, more specifically one or more of an inner diameter at a predefined position above the sample tube's bottom and an inner diameter at a predefined position below the sample tube's upper edge, and an inner diameter of a cap of the sample tube; an outer diameter of the sample tube, specifically one or more of an outer diameter of a sample tube body of the sample tube, more specifically one or more of an outer diameter at a predefined position above the sample tube's bottom and an outer diameter at a predefined position below the sample tube's upper edge, and an outer diameter of a cap of the sample tube; a shape of at least a part of the sample tube, specifically one or more of a shape of a tube bottom of the sample tube and a shape of a cap of the sample tube; a geometry of a tube bottom of the sample tube, specifically one or more of a radius of a round bottom of the sample tube and a length of a flat bottom of the sample tube; a geometry of a thread of the sample tube, specifically one or more of an outer diameter of a thread of the sample tube, a length of a thread of the sample tube and a number of threads of a thread of the sample tube; an angle of the sample tube's body, specifically one or more of a left angle of the sample tube's body and a right angle of the sample tube's body.
Embodiment 24: The method according to any one of the two preceding embodiments, wherein the item of color identification information comprises at least one item of color information on a cap of the sample tube.
Embodiment 25: The method according to any one of the preceding embodiments, wherein the second item of identification information comprises at least one of: an item of material identification information; an item of functional identification information; an item of geometrical identification information.
Embodiment 26: The method according to the preceding embodiment, wherein the item of material identification information comprises at least one of: information on a material of a tube body of the sample tube; information on a material of a cap of the sample tube.
Embodiment 27: The method according to any one of the two preceding embodiments, wherein the item of functional identification information comprises information on a cap sealing type of a cap of the sample tube.
Embodiment 28: The method according to any one of the three preceding embodiments, wherein the item of geometrical identification information comprises information on a tube geometry of at least a part of the sample tube, specifically on one or more of a geometry of a tube bottom of the sample tube and a cap of the sample tube.
Embodiment 29: The method according to any one of the preceding embodiments, wherein the mobile device comprises at least one processor.
Embodiment 30: The method according to the preceding embodiment, wherein the processor is adapted to perform step ii) and optionally step iii).
Embodiment 31: The method according to any one of the two preceding embodiments, wherein the processor is adapted to automatically initiate the capturing of the image in step i), specifically by recognizing if the sample tube is visible in a field of view of the camera.
Embodiment 32: The method according to any one of the preceding embodiments, wherein step iv) at least partially is performed by a backend server remote from the mobile device, specifically by a cloud server.
Embodiment 33: The method according to the preceding embodiment, wherein the method further comprises transmitting data obtained in one or more of steps i), ii), and iii) from the mobile device to the backend server.
Embodiment 34: The method according to any one of the preceding embodiments, wherein data obtained in one or both of steps i) and step iii) are recorded via a software application running on the mobile device.
Embodiment 35: An assessment system for assessing the suitability of at least one sample tube for use in a laboratory automation system, comprising at least one mobile device having at least one camera, wherein the mobile device is configured, specifically by software programming, for capturing at least one image of at least a part of the sample tube by using the camera, wherein the assessment system is configured, specifically by software programming, for performing the method according to any one of the preceding embodiments.
Embodiment 36: The assessment system according to the preceding embodiment, wherein the assessment system further comprises at least one backend server remote from the mobile device, specifically a cloud server, wherein the backend server is configured for data exchange with the mobile device, wherein the backend server is configured, specifically by software programming, for at least partially performing step iv).
Embodiment 37: The assessment system according to any one of the preceding embodiments referring to an assessment system, wherein the mobile phone is further configured, specifically by software programming, for performing at least one of steps ii) and iii).
Embodiment 38: A mobile device, specifically for use in an assessment system according to any one of the preceding embodiments referring to an assessment system, wherein the mobile device is configured, specifically by software programming, for performing at least steps i), ii) and iii) of the method according to any one of the preceding method embodiments.
Embodiment 39: The mobile device according to the preceding embodiment, wherein the mobile device is further configured for transmitting data obtained in one or more of steps i), ii), and iii) to a backend server.
Embodiment 40: A computer program comprising instructions which, when the program is executed by at least one processor of the assessment system according to any one of the preceding embodiments referring to an assessment system, cause the processor to carry out the method according to any one of the preceding method embodiments.
Embodiment 41: A computer-readable storage medium comprising instructions which, when executed by at least one processor of the assessment system according to any one of the preceding embodiments referring to an assessment system, cause the processor to carry out the method according to any one of the preceding method embodiments.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows embodiments of an assessment system, of a mobile device and of a laboratory automation system;
- Figures 2 and 3: show flow charts of different embodiments of a method of assessing the suitability of a sample tube for use in a laboratory automation system;
- Figure 4: shows an embodiment of a calibration element and of a sample tube;
- Figures 5a-c: show different register cards of an embodiment of a graphical user interface for retrieving at least one second item of identification information; and
- Figure 6: shows an embodiment of a graphical user interface displaying the result of the method.

### Detailed description of the embodiments

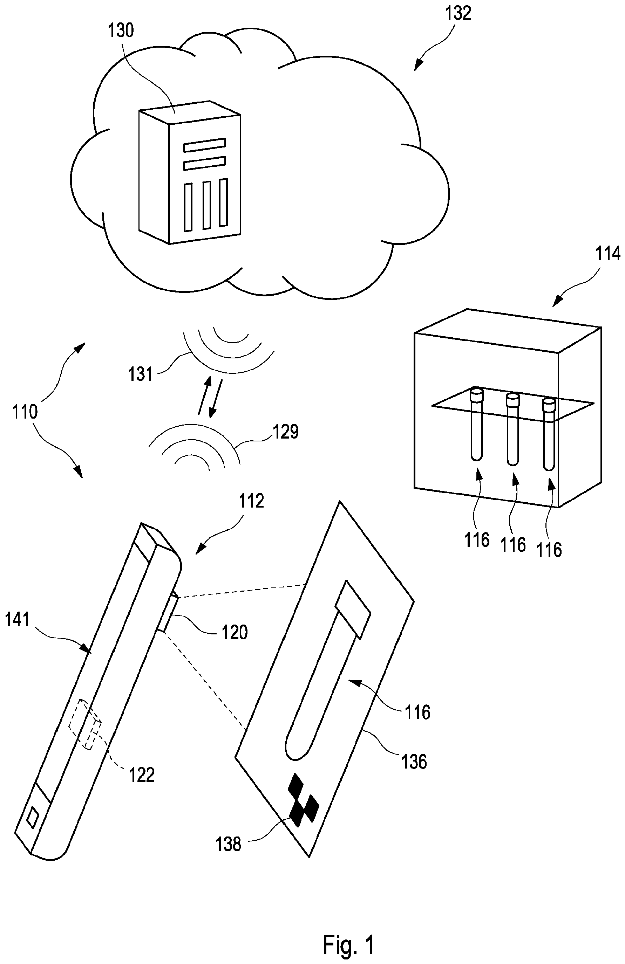

In Figure 1, embodiments of an assessment system 110, of a mobile device 112 and of a laboratory automation system 114 according to the present invention are shown in a schematic view.

The assessment system 110 comprises the at least one mobile device 112 and is configured, specifically by software programming, for performing a method 118 of assessing the suitability of at least one sample tube 116 for use in the laboratory automation system 114. The assessment system 110 further comprises at least one backend sever device 130, such as at least one cloud server 132, as will be outlined in further detail below. The mobile device 112 has at least one camera 120 and may comprise at least one processor 122.

The assessment system 110 may further comprise at least one calibration element 136. The calibration element 136 may comprise at least one geometrical reference marker 138 and/or at least one color reference, as will be explained in further detail below.

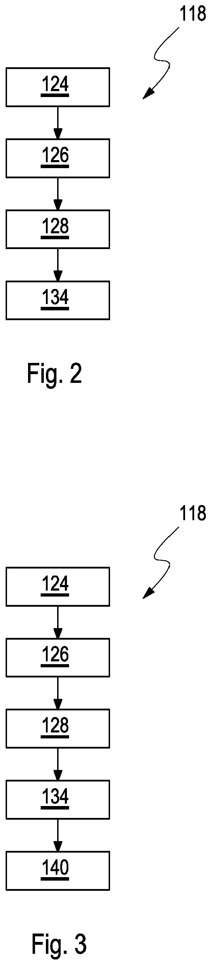

The assessment system 110 is configured for performing a method of assessing the suitability of the at least one sample tube 116 for use in the laboratory automation system 114. In Figure 2, a flow chart of an exemplary embodiment of the method is shown, wherein the method is denoted by reference number 118. The method 118 comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method 118 may comprise additional method steps that are not listed. The method steps of the method 118 are the following:
i) (denoted with reference number 124) capturing at least one image of at least a part of the sample tube 116 by using the camera 120 of the mobile device 112;
ii) (denoted with reference number 126) obtaining at least one first item of identification information of the sample tube 116 by analyzing the image;
iii) (denoted with reference number 128) retrieving at least one second item of identification information of the sample tube 116, specifically by the mobile device 112; and
iv) (denoted with reference number 134) evaluating the first and second items of identification information and assigning the sample tube 116 to at least one suitability category.

The image captured in step i) 124 of the method 118 may comprise at least a part of the calibration element 136. In step ii) 126, the first item of identification information may comprise at least one of an item of geometrical identification information and/or an item of color identification information. The calibration element 136 may be used to obtain the item of geometrical identification information and/or the item of color identification information of at least a part of the sample tube 116. For example, the geometrical item of identification information may comprise at least one of: a height of the sample tube 116; a width of the sample tube 116; an inner diameter of the sample tube 116; an outer diameter of the sample tube 116 and/or a shape of the sample tube 116. The item of color identification information may comprise, for example, at least one item of color information on a cap of the sample tube 116. Further, the first item of identification information is obtained by image analysis. For example, the image analysis may be implemented by a computer vision process. Other image analysis steps or processes may be used in addition or alternatively.

In step iii) 128 of the method 118, a second item of identification information is retrieved, whereby the second item of identification information may comprise information other than information obtained via image analysis. The second item of identification information may comprise at least one of: an item of material identification information; an item of functional identification information and/or an item of geometrical identification information. The item of material identification information may comprise information on a material of a tube body and/or of a cap of the sample tube 116. The item of functional identification information may comprise information on a cap sealing type of the cap of the sample tube 116. The item of geometrical identification information may comprise information on a geometry of at least a part of the sample tube 116, specifically on one or more of a geometry of a tube bottom and/or of a cap of the sample tube 116.

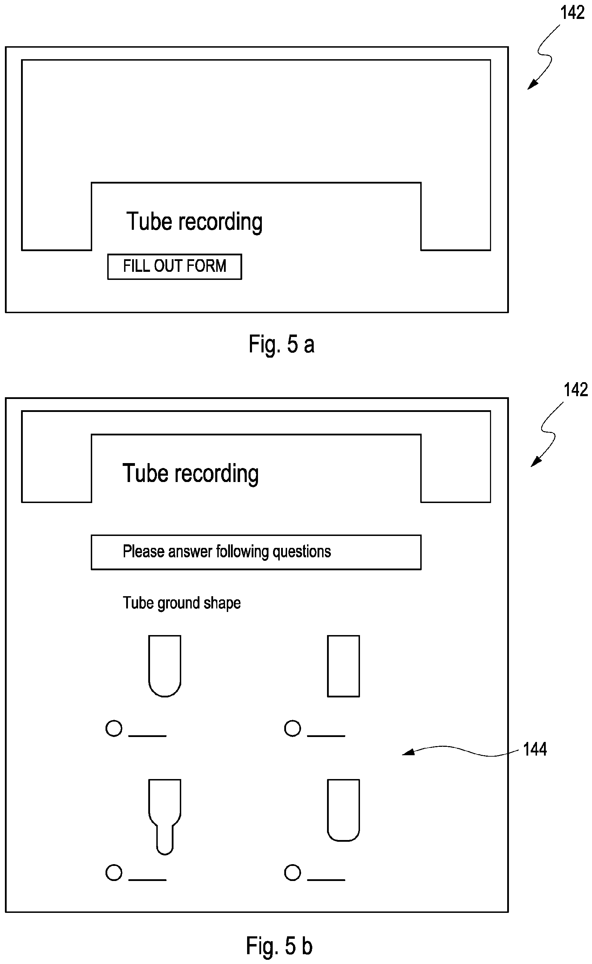

Further, the second item of identification information may be retrieved via an interface of the mobile device 112, wherein the interface may comprise at least one of a data interface and/or a user interface, specifically a graphical user interface. The mobile device 112 may prompt a user to input the second item of identification information. For example, the second item of identification information may be retrieved by a user input, specifically via a manual user input. An exemplary embodiment of different register cards of the graphical user interface is shown in Figures 5a-c and will be described in further detail below.

In step iv) 134, the first and second items of identification information are evaluated and the sample tube 116 is assigned to at least one suitability category. Step iv) 134 may comprise combining the first and second items of identification information to obtain at least one combined item of identification information of the sample tube 116, wherein, in step iv) 134, the combined item of identification information may be evaluated. The combined item of identification information may comprise a serial number or an article number of the sample tube 116. Additionally or alternatively, the at least one combined item of identification information may be or may comprise a vector and/or an array of items of information, such as an array comprising both the at least one first item of identification information and the at least one second item of identification information. The combined item of identification information may then be used for further processing, such as for identifying the sample tube 116 and/or for determining the suitability category. Further, step iv) 134 may be performed by using an algorithm, specifically a machine learning algorithm, more specifically a machine learning classifier. The assigning of the sample tube 116 to at least one suitability category may be performed by using at least one sample tube database. The suitability category may indicate whether or not the sample tube 116 is of a type already listed in the sample tube database and furthermore, whether or not the sample tube 116 is suitable for being used with the laboratory automation system 114.

As outlined above, the processor 122 of the mobile device 112 may be adapted to initiate capturing the image in step i) 124 and to perform step ii) 126 and optionally step iii) 128. Further, step iv) 134 may be at least partially performed by the backend server 130 remote from the mobile device 112, specifically by the cloud server 132. Therefore, the method 118 may comprise transmitting the data obtained in one or more of steps i) 124, ii) 126 or iii) 128 from the mobile device 112 to the backend server 130.

In Figure 3, a flow chart of a further embodiment of a method of assessing the suitability of the at least one sample tube 116 for use in the laboratory automation system 114 is shown. The embodiment widely corresponds to the embodiment of Figure 2. Further, however, as illustrated by Figure 3, the method 118 may comprise an additional step v) 140, in which the suitability category is provided via at least one interface, specifically via at least one interface of the mobile device 112 such as a user interface, more specifically via at least one display of the mobile device 112.

Turning back to Figure 1, the mobile device 112 is configured, specifically by software programming, for performing at least steps i) 124, ii) 126 and iii) 128 of the method 118, such as according to any one of Figures 2 or 3. In step i) 124 of the method 118, the mobile device 112 is used to capture at least one image of at least a part of the sample tube 116 by using the camera 120 of the mobile device 112. The processor 122 of the mobile device 112 may be adapted to automatically initiate the capturing of the image, specifically by recognizing if the sample tube 116 is visible in a field of view of the camera 120. As example, a "live" image of a scene visible in the field of view of the camera 120 may be evaluated, e.g. continuously, in order to detect the presence of the sample tube 116. However, another way of triggering the image capture is also feasible, such as a manual triggering. Further, the processor 122 may be adapted to perform step ii) 126 and optionally step iii) 128 of the method 118.

The mobile device 112 may further be configured for transmitting the data obtained in one or more of steps i) 124, ii) 126 and iii) 128 from the mobile device 112 to a backend server 130 remote from the mobile device 112. For this purpose, as an example, at least one interface 129 of the mobile device 112 may be used, such as a wireless interface, communicating with at least one interface 131 of the backend server 130. Further, the backend server 130 may be configured, specifically by software programming, for at least partially performing step iv) 134.

Further, step i) 124 of the method 118 may comprise using the at least one calibration element 136, wherein the image may comprise at least a part of the calibration element 136. The calibration element 136 may comprise at least one geometrical reference marker 138 and/or at least one color reference.

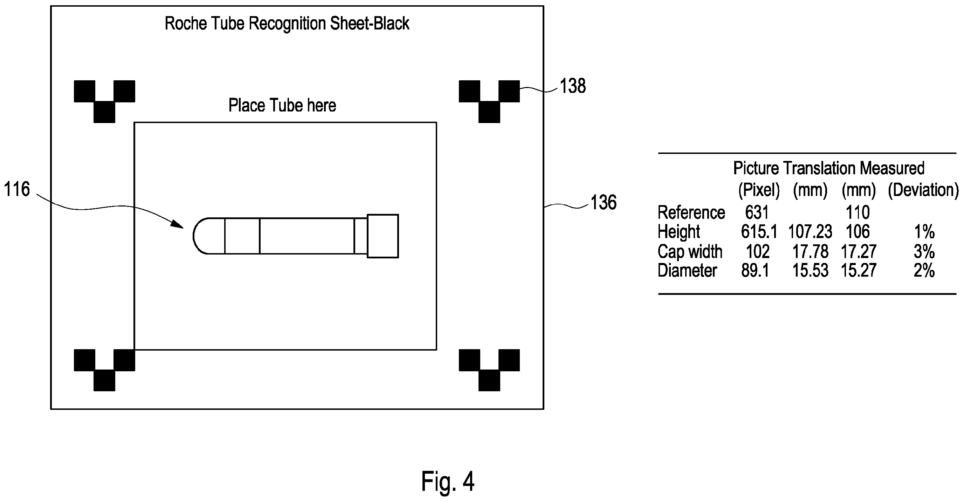

Figure 4 shows an exemplary embodiment of the calibration element 136 and of the sample tube 116. The calibration element 136 may comprise one or more of a geometrical reference marker 138 and/or a color reference. The geometrical reference marker 138 may have known geometrical properties and may be used to calibrate one or more of geometrical and/or dimensional information derived from the image. The color reference may have known color properties and may be used to calibrate color information derived from the image. The sample tube 116 may be placed onto the calibration element 136 while performing step i) 124 of the method 118. Additionally or alternatively, the calibration element 136 may also be placed next to the sample tube 116, such that both the sample tube 116 and the calibration element 136 may be at least partially visible in the image. The calibration element 136 may have a high contrast compared to the sample tube. Specifically, the calibration element 136 may have a dark background if the sample tube comprises bright colors, or vice versa.

As outlined above, display 141 may be used as a graphical user interface, e.g. by allowing displaying information and/or by allowing for a user to input data via the display 141, such as via a touch screen. Additionally or alternatively, the mobile device 112 may comprise further user interfaces. The method 118, as outlined above, may be computer-implemented, such as at least partially by an application or App running on the mobile device 112 and, additionally, software running on the cloud server 132.

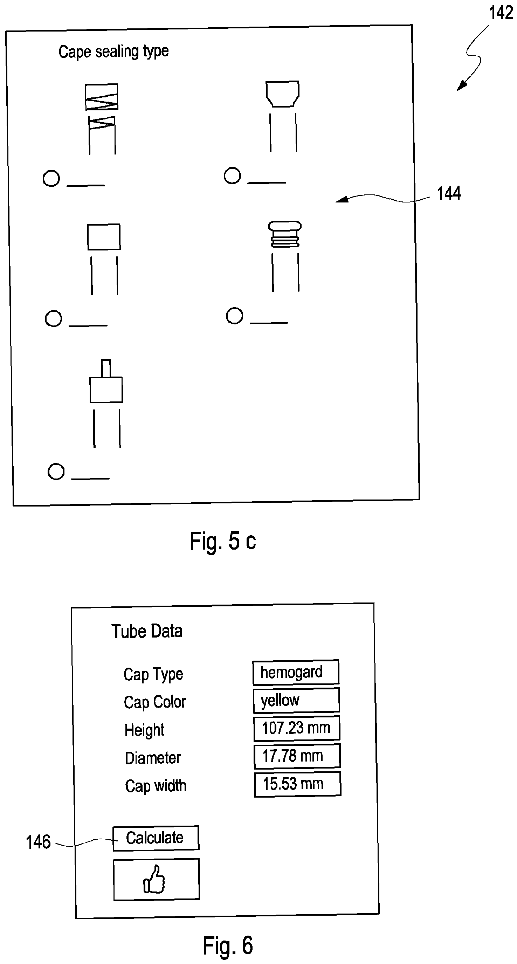

In Figures 5a-c, different register cards of an exemplary embodiment of the graphical user interface are illustrated, as may be generated by the App executed by the processor 122 of the mobile device 112. As outlined above, in step iii) 128 of the method 118, the second item of identification information is at least partially retrieved via a graphical user interface. For example, the second item of identification information may at least partially be retrieved via a software application running on the mobile device 112, the software application requesting a user of the mobile device 112 to input the second item of identification information, specifically via an input menu 142, more specifically via having the user select from a plurality of predetermined items 144. The plurality of predetermined items 144 may comprise one or more of: a material of a tube body of the sample tube 116; a material of a cap of the sample tube 116; a cap sealing type of a cap of the sample tube 116; a geometry of a tube bottom of the sample tube 116; a geometry of a cap of the sample tube 116. Thus, in Figure 5a, the mobile device 112 may start the request to input the second item of identification, by requesting the user to fill out a form as shown in the subsequent images. In Figures 5b and 5c, input menus 142 are shown, requesting the user to select from the plurality of predetermined items 144. Figure 5b shows the request for selection from a plurality of predetermined tube bottoms, also referred to as tube ground shapes. Figure 5c shows the request for selection from a plurality of predetermined cap sealing types.

Method 118 may generally comprise showing or displaying at least one result, such as the at least one suitability category, such by using the display 141 of the mobile device 112.

Thus, Figure 6 shows an exemplary embodiment of a graphical user interface displaying the result of the method 118. The result of the method 118 may comprise the suitability category which is assigned to the sample tube 116 in step iv) 134 of the method 118. The suitability category may comprise at least one category from the group consisting of: "known and compatible", "unknown, but compatible" and "not compatible". As outlined above, the suitability category may be provided via at least one interface, specifically via at least one interface of the mobile device 112, more specifically via at least one display of the mobile device 112. For example, the software application running on the mobile device 112 may further be configured for providing the suitability category via the at least one interface of the mobile device 112, such as via the at least one graphical user interface, specifically for displaying the suitability on at least one display of the mobile device 112. Other options are also feasible. In Figure 6, as an example, firstly, the tube data of sample tube 116 are displayed, such as the cap type, the cap color, the height, the diameter, and the cap width. This summary may allow for the user to correct data or add missing data. Further, an interactive button 146 may be displayed, allowing for a user to actively start the evaluation, based on the tube data listed above. Having started the evaluation, at least one result may be shown, such as the suitability category. Thus, as an example shown in Figure 6, the "thumbs-up" symbol may indicate that the sample tube 116 is "known and compatible" for use with the laboratory automation system 114. Other symbols and/or text may be used for displaying the result. Further, besides the at least one suitability category, additional information may also be displayed.

### List of reference numbers

- 110: assessment system
- 112: mobile device
- 114: laboratory automation system
- 116: sample tube
- 118: assessment method
- 120: camera
- 122: processor
- 124: step i)
- 126: step ii)
- 128: step iii)
- 128: interface of the mobile device
- 130: backend server
- 131: interface of the backend server
- 132: cloud server
- 134: step iv)
- 136: calibration element
- 138: geometrical reference marker
- 140: step v)
- 141: display
- 142: input menu
- 144: plurality of predetermined items
- 146: button

## Claims

1. A method of assessing the suitability of at least one sample tube (116) for use in a laboratory automation system (114), comprising:
i) capturing at least one image of at least a part of the sample tube (116) by using a camera (120) of a mobile device (112);
ii) obtaining at least one first item of identification information of the sample tube (116) by analyzing the image;
iii) retrieving at least one second item of identification information of the sample tube (116); and
iv) evaluating the first and second items of identification information and assigning the sample tube (116) to at least one suitability category.

2. The method according to the preceding claim, wherein the second item of identification information is at least partially retrieved by a user input.

3. The method according to the preceding claim, wherein the mobile device (112) prompts the user to input the second item of identification information.

4. The method according to any one of the preceding claims, further comprising:
v) providing the suitability category via at least one display (141) of the mobile device (112).

5. The method according to the preceding claim, wherein step i) (124) comprises using at least one calibration element (136), wherein the image comprises at least a part of the calibration element (136).

6. The method according to any one of the preceding claims, wherein step iv) (134) is performed by using a machine learning algorithm.

7. The method according to any one of the preceding claims, wherein the assigning of the sample tube (116) to at least one suitability category in step iv) (134) is performed by using at least one sample tube database.

8. The method according to any one of the preceding claims, wherein the first item of identification information comprises at least one of: an item of geometrical identification information; an item of color identification information.

9. The method according to any one of the preceding claims, wherein the second item of identification information comprises at least one of: an item of material identification information; an item of functional identification information; an item of geometrical identification information.

10. The method according to any one of the preceding claims, wherein step iv) (134) at least partially is performed by a backend server (130) remote from the mobile device (112).

11. An assessment system (110) for assessing the suitability of at least one sample tube (116) for use in a laboratory automation system (114), comprising at least one mobile device (112) having at least one camera (120), wherein the mobile device (112) is configured for capturing at least one image of at least a part of the sample tube (116) by using the camera (120), wherein the assessment system (110) is configured for performing the method according to any one of the preceding claims.

12. The assessment system (110) according to the preceding claim, wherein the assessment system (110) further comprises at least one backend server (130) remote from the mobile device (112), wherein the backend server (130) is configured for data exchange with the mobile device (112), wherein the backend server (130) is configured for at least partially performing step iv) (134).

13. A mobile device (112), wherein the mobile device (112) is configured for performing at least steps i) (124), ii) (126) and iii) (128) of the method according to any one of the preceding method claims.

14. The mobile device (112) according to the preceding claim, wherein the mobile device (112) is further configured for transmitting data obtained in one or more of steps i) (124), ii) (126), and iii) (128) to a backend server (130).

15. A computer-readable storage medium comprising instructions which, when executed by at least one processor (122) of the assessment system (110) according to any one of the preceding claims referring to an assessment system (110), cause the processor (122) to carry out the method according to any one of the preceding method claims.
